# FASCICULE DE BREVET EUROPEEN

(11) **EP 0 974 342 B1**
(45) Date de publication et mention de la délivrance du brevet: **12.09.2001**
(21) Numéro de dépôt: 99401697.0
(22) Date de dépôt: 07.07.1999
(51) Int. Cl.: A61K 7/50, A61K 7/48

(54) **Composition cosmétique aqueuse transparente à une seule phase**
Einphasige,transparente wässrige Zusammensetzung
Single phase transparent aqueous composition

(30) Priorité: 21.07.1998 FR 9809301
(43) Date de publication de la demande: 26.01.2000
(73) Titulaire: L'OREAL, 75008 Paris (FR)
(72) Inventeur: Bui-Bertrand, Lien, 91600 Savigny/S/Orge (FR); Carrel, Marie-Laure, 75015 Paris (FR)
(74) Mandataire: Rasson, Catherine

(56) Documents cités:
- WO-A-95/16661
- WO-A-95/34540
- FR-A- 2 358 148

## Description

L'invention a pour objet une composition cosmétique liquide aqueuse transparente à une seule phase, contenant au moins un métabisulfite et au moins un para-hydroxybenzoate d'alkyle (paraben), et à l'utilisation de la dite composition pour le nettoyage et/ou le démaquillage, en douceur, de la peau et/ou des yeux. Elle a aussi pour objet un procédé pour solubiliser un para-hydroxybenzoate d'alkyle dans une composition cosmétique aqueuse, consistant à ajouter au moins un métabisulfite.

Les compositions liquides aqueuses transparentes à une seule phase (monophasée) sont des compositions ne comportant pas de phase huileuse et constituant des lotions de nettoyage et de démaquillage. Ces lotions sont couramment utilisées dans le domaine cosmétique, notamment pour parfaire le démaquillage de la peau et/ou des yeux après l'utilisation d'un lait. Elles contiennent une grande quantité d'eau et constituent par exemple des toniques et des démaquillants pour les yeux.

Ces compositions liquides aqueuses monophasées contiennent des conservateurs, notamment des para-hydroxybenzoates d'alkyle encore appelés parabens. Ces parabens et notamment le méthylparaben, largement utilisés en cosmétique pour leurs propriétés antifongiques, présentent l'inconvénient d'être peu solubles en milieu aqueux. Or si leur solubilisation n'est pas totale, les parabens ont tendance à recristalliser, ce qui se traduit par la formation de particules blanches. De telles particules blanches rendent rédhibitoires l'aspect visuel et la qualité sensorielle d'une composition transparente. En outre, pour remplir pleinement leur rôle de conservateurs dans la phase aqueuse, lieu de contamination, les parabens doivent être parfaitement solubilisés dans la dite phase aqueuse.

Il est donc essentiel que la solubilisation des parabens dans les compositions aqueuses, et notamment les compositions aqueuses transparentes, soit totale pour atteindre la plus grande efficacité possible et aussi pour obtenir une transparence parfaite de la composition.

Pour favoriser la solubilisation des parabens, une solution consiste à ajouter à la composition aqueuse un alcool primaire tel que l'éthanol, un polyol tel qu'un glycol, ou un tensioactif. Toutefois, l'addition d'alcool primaire est à éviter notamment dans les lotions pour le visage car l'éthanol peut avoir un caractère irritant. Par ailleurs, l'addition d'une trop grande quantité de glycols confère à une composition liquide telle qu'une lotion, un caractère poisseux et collant. De plus, on cherche à éviter l'emploi de trop grande quantité de tensioactifs du fait de leur caractère irritant pour la peau et les yeux et notamment chez les sujets sensibles. En outre, certains tensioactifs anioniques et non ioniques sont incompatibles avec les parabens dont ils inhibent l'activité.

Il subsiste donc le besoin de disposer d'une composition cosmétique liquide aqueuse à une seule phase, n'ayant pas les inconvénients de celles de l'art antérieur et qui soit parfaitement transparente grâce à une parfaite solubilisation des parabens qu'elle contient.

Or, la demanderesse a constaté avec étonnement que les métabisulfites et notamment le métabisulfite de sodium permettent d'obtenir des compositions aqueuses tout à fait transparentes, dans lesquelles les parabens et notamment le méthylparaben sont parfaitement solubilisés. Le produit final a une bonne stabilité et une protection microbiologique conservée.

La présente invention a donc pour objet une composition cosmétique liquide aqueuse transparente à une seule phase, caractérisée par le fait qu'elle contient au moins un para-hydroxybenzoate d'alkyle, le groupe alkyle ayant de 1 à 6 atomes de carbone, au moins 90 % en poids d'eau par rapport au poids total de la composition et au moins un métabisulfite, une quantité de métabisulfite étant d'au plus 0,02 % en poids par rapport au poids total de la composition.

Le mot « transparente » signifie qu'au travers de la bouteille contenant la composition, on peut distinguer les caractères imprimés sur une page de journal placée derrière cette bouteille. Ce terme signifie également qu'un échantillon de 10 cm d'épaisseur de la composition a une transmission maximum de la lumière d'au moins 4% dans n'importe quelle longueur d'onde comprise entre 200 nm et 800 nm.

On entend par composition liquide une composition contenant une forte proportion d'eau et ayant une densité égale à environ 1, c'est-à-dire allant d'environ 0,99 à 1,01. La composition de l'invention contient au moins 90 % en poids d'eau et de préférence au moins 95 % en poids d'eau par rapport au poids total de la composition. La quantité d'eau peut aller jusqu'à 99,9 % en poids par rapport au poids total de la composition.

Rien ne laissait supposer que le métabisulfite de sodium connu pour ses propriétés anti-oxydantes était susceptible d'être utilisé avec succès, même en une petite quantité, pour solubiliser les parabens, ceci permettant d'obtenir à la fois une bonne conservation de la composition cosmétique et une parfaite transparence, ainsi qu'une excellente tolérance, grâce à l'utilisation de quantités de métabisulfite, suffisamment petites (égales ou inférieures à 0,02 %) pour ne pas être irritantes, ce qui est particulièrement important pour une utilisation cosmétique et notamment pour le démaquillage des yeux.

Aussi, l'invention a encore pour objet à un procédé pour solubiliser un para-hydroxybenzoate d'alkyle dans une composition cosmétique aqueuse, consistant à ajouter au moins un métabisulfite.

Le métabisulfite est généralement un métabisulfite de métal alcalin et de préférence le métabisulfite de sodium. Il est de préférence présent en une quantité efficace pour assurer le résultat escompté et peut être par exemple présent dans la composition selon l'invention en une quantité allant de 0,001 à 0,02 %, de préférence de 0,002 à 0,01 % en poids par rapport au poids total de la composition.

Dans le para-hydroxybenzoate d'alkyle, le radical alkyle comprend de 1 à 6 atomes de carbone et de préférence de 1 à 4 atomes de carbone. Comme para-hydroxybenzoate d'alkyle, on peut notamment citer le para-hydroxybenzoate de méthyle (méthylparaben), le para-hydroxybenzoate d'éthyle (éthylparaben), le para-hydroxybenzoate de propyle (propylparaben), le para-hydroxybenzoate de butyle (butylparaben) et le para-hydroxybenzoate d'isobutyle (isobutylparaben).

Dans la composition de l'invention, on utilise de préférence le para-hydroxybenzoate de méthyle, seul ou en mélange avec un ou plusieurs autres parabens et/ou avec un autre conservateur tel que le phénoxyéthanol. On peut par exemple utiliser le mélange de méthylparaben, éthylparaben, propylparaben et butylparaben, vendu sous la dénomination NIPASTAT par la société NIPA, ou le mélange de phénoxyéthanol, méthylparaben, éthylparaben, propylparaben et butylparaben, vendu sous la dénomination PHENONIP par la société NIPA.

Le para-hydroxybenzoate d'alkyle ou le mélange de para-hydroxybenzoates d'alkyle est utilisé dans la composition selon l'invention en une quantité adaptée au but recherché, c'est-à-dire une bonne conservation microbiologique de la composition. Cette quantité peut aller par exemple de 0,01 à 1 % et de préférence de 0,05 à 0,5 % en poids par rapport au poids total de la composition.

De préférence, la composition selon l'invention est exempte d'alcool monohydrique en C₁-C₃, et notamment d'éthanol, dont on cherche à éviter l'emploi du fait de son caractère irritant et inflammable. Elle est de même de préférence exempte d'agent gélifiant.

La composition de l'invention peut comprendre un ou plusieurs polyols et notamment des glycols en une quantité telle qu'elle ne confère pas de caractère collant à la composition finale. Parmi les polyols utilisables dans la composition selon l'invention, on peut citer notamment le propylène glycol, le butylène glycol, la glycérine, l'hexylène glycol, les polyéthylène glycols tels que le PEG-8, le dipropylène glycol et leurs mélanges. La quantité de polyol(s) dans la composition de l'invention va en général de 0,1 à 9,5 % et de préférence de 1 à 5 % en poids par rapport au poids total de la composition.

La composition liquide aqueuse de l'invention peut éventuellement comprendre un tensioactif dont la présence permet d'améliorer le démaquillage. Ce tensioactif est en général présent en une quantité allant de 0,01 à 5 %, et encore plus préférentiellement 0,05 à 2 % en poids par rapport au poids total de la composition.

Comme tensioactif susceptible d'être utilisé dans la composition de l'invention, on peut citer le Sodium Laureth Sulfate tel que le produit "Texapon ASV" commercialisé par la Société HENKEL, le Disodium Cocoamphodiacétate tel que le produit "Miranol 2CM" commercialisé par la Société RHODIA Chimie, les Polysorbates, et les Poloxamer tels que les produits commercialisés par ICI sous le nom de Symperonic.

Dans la composition de l'invention, l'eau utilisée peut être de l'eau pure déminéralisée mais aussi de l'eau minérale et/ou de l'eau thermale, c'est-à-dire que l'eau de la composition peut être en partie ou totalement constituée par une eau choisie parmi les eaux minérales et/ou thermales. En général, une eau minérale est propre à la consommation, ce qui n'est pas toujours le cas d'une eau thermale. Chacune de ces eaux contient, entre autre, des minéraux solubilisés et/ou des oligo-éléments. Ces eaux sont connues pour être employées à des fins de traitement spécifique selon les oligo-éléments et les minéraux particuliers qu'elles contiennent, tel que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon l'invention peut, par exemple, être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

Les compositions cosmétiques de l'invention sont destinées à une application topique et comprennent de manière appropriée un milieu physiologiquement acceptable. Par milieu physiologiquement acceptable, on entend un milieu compatible avec la peau, les muqueuses (y compris l'intérieur des paupières et les lèvres), les ongles et/ou les fibres kératiniques (cheveux et cils). Ces compositions peuvent, en outre, contenir des adjuvants habituels dans le domaine cosmétique, tels que les antioxydants, les parfums, les filtres, les matières colorantes, les actifs hydrophiles ou lipophiles. La nature des adjuvants et leurs quantités doivent être telles qu'elles ne modifient pas les propriétés de la composition selon l'invention. Les quantités de ces adjuvants sont celles classiquement utilisées dans le domaine cosmétique et par exemple de 0,001 à 5 % du poids total de l'émulsion.

Les compositions de l'invention constituent des lotions particulièrement adaptées au nettoyage et/ou au démaquillage de la peau et/ou des yeux.

Aussi, l'invention a encore pour objet l'utilisation cosmétique de la composition telle que définie ci-dessus pour le nettoyage et/ou le démaquillage de la peau et/ou des yeux.

Les exemples suivants sont donnés à titre d'illustration de l'invention et n'ont pas de caractère limitatif. Toutes les quantités sont données en pourcentage en poids de matière active par rapport au poids total de la composition. Les matières premières sont données en nom CTFA.

### Exemple 1 : lotion pour le nettoyage du visage et des yeux

| | |
|---|---|
| Glycérine | 3 % |
| Sodium metabisulfite | 0,002 % |
| Methylparaben | 0,1 % |
| Eau déminéralisée | qsp 100 % |

La composition est préparée en mélangeant le méthylparaben et la glycérine à 60°C, puis en introduisant le mélange, sous forte agitation et à température ambiante, dans l'eau contenant le métabisulfite de sodium.

### Exemple 2 : lotion pour le nettoyage du visage et des yeux

| | |
|---|---|
| PEG-8 | 2 % |
| Methylparaben | 0,1 % |
| Sodium metabisulfite | 0,005 % |
| Eau déminéralisée | qsq 100 % |

La composition est préparée en mélangeant le méthylparaben et l'eau à 80°C, puis en additionnant au mélange refroidi, le PEG-8 et le métabisulfite de sodium, sous agitation et à température ambiante.

### Exemple 3 : lotion pour le nettoyage du visage et des veux

| | |
|---|---|
| Methylparaben | 0,1 % |
| Sodium metabisulfite | 0,005 % |
| PEG-8 | 2 % |
| Glycérine | 3 % |
| Eau de bleuet | 1 % |
| Eau déminéralisée | qsq 100 % |

La composition est préparée en mélangeant le méthylparaben et la glycérine à 60°C, puis en introduisant le mélange dans l'eau sous forte agitation à la température ambiante. Lorsque la solution est limpide, on ajoute le métabisulfite de sodium, le PEG-8 et l'eau de bleuet, chaque ingrédient étant introduit successivement sous agitation et à température ambiante.

## Revendications

1. Composition cosmétique liquide aqueuse transparente à une seule phase, **caractérisée par** le fait qu'elle contient au moins un para-hydroxybenzoate d'alkyle, le groupe alkyle ayant de 1 à 6 atomes de carbone, au moins 90 % en poids d'eau par rapport au poids total de la composition et au moins un métabisulfite, la quantité de métabisulfite étant d'au plus 0,02 % en poids par rapport au poids total de la composition.

2. Composition selon la revendication 1, **caractérisée en ce qu'**elle a une densité allant de 0,99 à 1,01.

3. Composition selon la revendication 1 ou 2, **caractérisée en ce qu'**elle comprend de 95 à 99,9 % en poids d'eau par rapport au poids total de la composition.

4. Composition selon l'une quelconque des revendications précédentes, **caractérisée en ce que** le métabisulfite est le métabisulfite de sodium.

5. Composition selon l'une quelconque des revendications précédentes, **caractérisée en ce que** le métabisulfite est présent en une quantité allant de 0,001 à 0,02 % et de préférence de 0,002 à 0,01 % en poids par rapport au poids total de la composition.

6. Composition selon l'une quelconque des revendications précédentes, **caractérisée en ce que** le para-hydroxybenzoate d'alkyle est le para-hydroxybenzoate de méthyle.

7. Composition selon la revendication précédente, **caractérisée en ce que** le para-hydroxybenzoate de méthyle est utilisé en mélange avec un ou plusieurs autres para-hydroxybenzoates d'alkyle et/ou avec un autre conservateur.

8. Composition selon l'une quelconque des revendications précédentes, **caractérisée en ce que** la quantité de para-hydroxybenzoate(s) d'alkyle va de 0,01 à 1 % en poids par rapport au poids total de la composition.

9. Composition selon l'une quelconque des revendications précédentes, **caractérisée en ce qu'**elle est exempte d'alcool monohydrique en C₁-C₃.

10. Composition selon l'une quelconque des revendications précédentes, **caractérisée en ce qu'**elle comprend au moins un polyol choisi parmi le propylène glycol, le butylène glycol, l'hexylène glycol, les polyéthylène glycols, la glycérine, le dipropylène glycol et leurs mélanges.

11. Composition selon la revendication précédente, **caractérisée en ce que** la quantité de polyol(s) va de 0,1 à 9,5 % en poids par rapport au poids total de la composition.

12. Composition selon l'une quelconque des revendications précédentes, **caractérisée en ce que** l'eau est en partie ou totalement constituée par une eau choisie parmi les eaux minérales et/ou thermales.

13. Composition selon l'une quelconque des revendications précédentes, **caractérisée en ce qu'**elle constitue une lotion de nettoyage et/ou de démaquillage de la peau et/ou des yeux.

14. Utilisation cosmétique de la composition selon l'une quelconque des revendications précédentes pour le nettoyage et/ou le démaquillage de la peau et/ou des yeux.

15. Procédé pour solubiliser un parahydroxybenzoate d'alkyle dans une composition cosmétique aqueuse, consistant à ajouter au moins un métabisulfite.

16. Procédé selon la revendication 15, **caractérisé en ce que** la composition comprend au moins 90 % d'eau.

17. Procédé selon la revendication 15 ou 16, **caractérisé en ce que** la composition comporte une seule phase.

18. Procédé selon l'une quelconque des revendications 15 à 17, **caractérisé en ce que** le métabisulfite est le métabisulfite de sodium.

## Patentansprüche

1. Transparente, wäßrige, flüssige kosmetische Zusammensetzung mit nur einer Phase, **dadurch gekennzeichnet, daß** sie mindestens ein Alkyl-p-hydroxybenzoat, wobei die Alkylgruppe 1 bis 6 Kohlenstoffatome aufweist, mindestens 90 Gew.-% Wasser, bezogen auf das Gesamtgewicht der Zusammensetzung, und mindestens ein Metabisulfit enthält, wobei der Mengenanteil des Metabisulfits höchstens 0,02 Gew.-%, bezogen auf das Gesamtgewicht der Zusammensetzung, beträgt.

2. Zusammensetzung nach Anspruch 1, **dadurch gekennzeichnet, daß** sie eine Dichte von 0,99 bis 1,01 aufweist.

3. Zusammensetzung nach Anspruch 1 oder 2, **dadurch gekennzeichnet, daß** sie 95 bis 99,9 Gew.-% Wasser, bezogen auf das Gesamtgewicht der Zusammensetzung, enthält.

4. Zusammensetzung nach einem der vorhergehenden Ansprüche, **dadurch gekennzeichnet, daß** das Metabisulfit das Natriummetabisulfit ist.

5. Zusammensetzung nach einem der vorhergehenden Ansprüche, **dadurch gekennzeichnet, daß** das Metabisulfit in einem Mengenanteil von 0,001 bis 0,02 Gew.-% und vorzugsweise 0,002 bis 0,01 Gew.-%, bezogen auf das Gesamtgewicht der Zusammensetzung, vorliegt.

6. Zusammensetzung nach einem der vorhergehenden Ansprüche, **dadurch gekennzeichnet, daß** das Alkyl-p-hydroxybenzoat das Methyl-p-hydroxybenzoat ist.

7. Zusammensetzung nach einem der vorhergehenden Ansprüche, **dadurch gekennzeichnet, daß** das Methyl-p-hydroxybenzoat im Gemisch mit einem oder mehreren weiteren Alkyl-p-hydroxybenzoaten und/oder einem anderen Konservierungsmittel verwendet wird.

8. Zusammensetzung nach einem der vorhergehenden Ansprüche, **dadurch gekennzeichnet, daß** der Mengenanteil des Alkyl-p-hydroxybenzoats oder der Alkyl-p-hydroxybenzoate im Bereich von 0,01 bis 1 Gew.-%, bezogen auf das Gesamtgewicht der Zusammensetzung, liegt.

9. Zusammensetzung nach einem der vorhergehenden Ansprüche, **dadurch gekennzeichnet, daß** sie keinen einwertigen C₁₋₃-Alkohol enthält.

10. Zusammensetzung nach einem der vorhergehenden Ansprüche, **dadurch gekennzeichnet, daß** sie mindestens ein Polyol enthält, das unter Propylenglykol, Butylenglykol, Hexylenglykol, Polyethylenglykolen, Glycerin, Dipropylenglykol und deren Gemischen ausgewählt ist.

11. Zusammensetzung nach dem vorhergehenden Anspruch, **dadurch gekennzeichnet, daß** die Menge des Polyols (der Polyole) im Bereich von 0,1 bis 9,5 Gew.-%, bezogen auf das Gesamtgewicht der Zusammensetzung, liegt.

12. Zusammensetzung nach einem der vorhergehenden Ansprüche, **dadurch gekennzeichnet, daß** das Wasser vollständig oder zum Teil aus Wasser besteht, das unter den Mineralwässern und/oder Thermalwässern ausgewählt ist.

13. Zusammensetzung nach einem der vorhergehenden Ansprüche, **dadurch gekennzeichnet, daß** sie eine Lotion zum Reinigen und/oder Abschminken der Haut und/oder der Augen darstellt.

14. Kosmetische Verwendung der Zusammensetzung nach einem der vorhergehenden Ansprüche zum Reinigen und/oder Abschminken der Haut und/oder der Augen.

15. Verfahren zur Solubilisierung eines Alkyl-p-hydroxybenzoats in einer wäßrigen kosmetischen Zusammensetzung, das darin besteht, mindestens ein Metabisulfit einzuarbeiten.

16. Verfahren nach Anspruch 15, **dadurch gekennzeichnet, daß** die Zusammensetzung mindestens 90 % Wasser enthält.

17. Verfahren nach Anspruch 15 oder 16, **dadurch gekennzeichnet, daß** die Zusammensetzung nur eine Phase aufweist.

18. Verfahren nach einem der Ansprüche 15 bis 17, **dadurch gekennzeichnet, daß** das Metabisulfit das Natriummetabisulfit ist.

## Claims

1. Single-phase transparent aqueous liquid cosmetic composition, **characterized in that** it comprises at least one alkyl para-hydroxybenzoate, the alkyl group having from 1 to 6 carbon atoms, at least 90% by weight of water with respect to the total weight of the composition and at least one metabisulphite, the amount of metabisulphite being at most 0.02% by weight with respect to the total weight of the composition.

2. Composition according to Claim 1, **characterized in that** it has a, density ranging from 0.99 to 1.01.

3. Composition according to Claim 1 or 2,
**characterized in that** it comprises 95% to 99.9% by weight of water with respect to the total weight of the composition.

4. Composition according to any one of the preceding claims, **characterized in that** the metabisulphite is sodium metabisulphite.

5. Composition according to any one of the preceding claims, **characterized in that** the metabisulphite is present in an amount ranging from 0.001 to 0.02% and preferably from 0.002 to 0.01% by weight with respect to the total weight of the composition.

6. Composition according to any one of the preceding claims, **characterized in that** the alkyl para-hydroxybenzoate is methyl para-hydroxybenzoate.

7. Composition according to the preceding claim, **characterized in that** methyl para-hydroxybenzoate is used as a mixture with one or more other alkyl para-hydroxybenzoates and/or with another preservative.

8. Composition according to any one of the preceding claims, **characterized in that** the amount of alkyl para-hydroxybenzoate(s) ranges from 0.01 to 1% by weight with respect to the total weight of the composition.

9. Composition according to any one of the preceding claims, **characterized in that** it is devoid of monohydric C₁-C₃ alcohol.

10. Composition according to any one of the preceding claims, **characterized in that** it comprises at least one polyol chosen from propylene glycol, butylene glycol, hexylene glycol, polyethylene glycols, glycerol, dipropylene glycol and their mixtures.

11. Composition according to the preceding claim, **characterized in that** the amount of polyol(s) ranges from 0.1 to 9.5% by weight with respect to the total weight of the composition.

12. Composition according to any one of the preceding claims, **characterized in that** the water is partially or entirely composed of a water chosen from mineral and/or thermal waters.

13. Composition according to any one of the preceding claims, **characterized in that** it constitutes a lotion for cleaning and/or removing make-up from the skin and/or eyes.

14. Cosmetic use of the composition according to any one of the preceding claims for cleaning and/or removing make-up from the skin and/or eyes.

15. Process for dissolving an alkyl para-hydroxybenzoate in an aqueous cosmetic composition which consists in adding at least one metabisulphite.

16. Process according to Claim 15, **characterized in that** the composition comprises at least 90% of water.

17. Process according to Claim 15 or 16, **characterized in that** the composition comprises a single phase.

18. Process according to any one of Claims 15 to 17, **characterized in that** the metabisulphite is sodium metabisulphite.
